# EUROPÄISCHE PATENTSCHRIFT

(11) **EP 0 586 918 B1**
(45) Veröffentlichungstag und Bekanntmachung des Hinweises auf die Patenterteilung: **02.01.1997**
(21) Anmeldenummer: 93112945.6
(22) Anmeldetag: 12.08.1993
(51) Int. Cl.: C07C 17/12, C07C 25/02

(54) **Verbessertes Verfahren zur Kernchlorierung von aromatischen Kohlenwasserstoffen**
Improved process for the nucleus chlorination of aromatic hydrocarbons
Procédé amélioré pour la chloration dans le noyau d'hydrocarbons aromatiques

(30) Priorität: 25.08.1992 DE 4228134
(43) Veröffentlichungstag der Anmeldung: 16.03.1994
(73) Patentinhaber: BAYER AG, 51368 Leverkusen (DE)
(72) Erfinder: Schrage, Heinrich, Dr., D-47800 Krefeld (DE); Fiege, Helmut, Dr., D-51373 Leverkusen (DE)

(56) Entgegenhaltungen:
- EP-A- 0 543 248
- FR-A- 2 377 988
- US-A- 3 000 975

## Beschreibung

Die Erfindung betrifft ein verbessertes Verfahren zur Kernchlorierung von aromatischen Kohlenwasserstoffen in Gegenwart einer Mischung von Friedel-Crafts-Katalysatoren und in Gegenwart von Co-Katalysatoren in flüssiger Phase.

Die Umsetzung von aromatischen Kohlenwasserstoffen in flüssiger Phase mit gasförmigem Chlor zu kernsubstituierten Chlorderivaten ist bekannt (siehe Ullmanns Encyclopädie der technischen Chemie, 4. Auflage, Band 9, Seiten 499 ff.). Man führt diese Chlorierung im allgemeinen in Gegenwart von Friedel-Crafts-Katalysatoren durch. Als Chlorierungsprodukt wird eine Mischung aus isomeren monochlorierten und polychlorierten Verbindungen erhalten. Bei Verwendung von FeCl₃ und Schwefel als Katalysator bzw. Co-Katalysator erhält man beispielsweise aus Toluol ein Gemisch aus Monochlortoluolen und Dichlortoluolen. In der Monochlortoluolfraktion sind die Hauptprodukte o-Chlortoluol und p-Chlortoluol neben einem geringen Anteil an m-Chlortoluol. Das Verhältnis voll o-Chlortoluol zu p-Chlortoluol beträgt etwa 1,1:1.

Da alle Monochlortoluole wertvolle Zwischenprodukte darstellen, wurde auch schon versucht, die Selektivität der Bildung einzelner Isomeren zu erhöhen. Von besonderer Bedeutung für die Zusammensetzung des Chlorierungsproduktes ist der verwendete Katalysator, wobei die bekannten Katalysatoren einen großen Spielraum ermöglichen. Eine Übersicht befindet sich in Ullmanns Encyclopedia of Industrial Chemistry, 5th Edition, Volume A6, S. 343. Häufig wird in technischem Maßstab FeCl₃ als Katalysator eingesetzt, das aufgrund seiner hohen Reaktivität bereits bei niedrigen Konzentrationen einen fast vollständigen Chlorumsatz gewährleistet. Nachteilig ist jedoch, daß relativ viel höher chlorierte Produkte gebildet werden. So enthält das Produkt der Chlorierung von Toluol mit FeCl₃ als Katalysator bei einem Chlorumsatz von 95 Mol-% bereits 9 Gew.-% unerwünschte Dichlortoluole.

Durch Zusatz von Schwefel läßt sich die Stufenselektivität erhöhen. Gleichzeitig erniedrigt sich aber das Verhältnis von o-Chlortoluol zu p-Chlortoluol auf 1,1:1.

Aus US 3 000 975 ist die Chlorierung von Toluol mit Titantetrachlorid, Zinntetrachlorid, Wolframhexachlorid und Zirkontetrachlorid als Katalysatoren bekannt. Das erreichbare Verhältnis von o-Chlortoluol zu p-Chlortoluol beträgt 3,3:1 und ist damit sehr hoch. Nachteilig bei diesem Verfahren ist jedoch die erforderliche hohe Katalysatorkonzentration von ca. 1 Gew.-% bezogen auf Toluol, die ca. 50 mal höher ist als die bei FeCl₃-Katalyse erforderliche Katalysatorkonzentration. Weiterhin ist nachteilig, daß ein hoher Anteil an o-Chlortoluol nur unter völligem Ausschluß von Eisen erreicht wird. Beispielsweise führt ein Zusatz von nur 10 ppm FeCl₃, eine Konzentration, die unter technischen Bedingungen schnell erreicht ist, zu dem gleichen niedrigen Verhältnis von o-Chlortoluol zu p-Chlortoluol und der gleichen schlechten Stufenselektiv wie sie mit reinem FeCl₃ als Katalysator erhalten wird.

Aus US 3 226 447 ist weiterhin die Chlorierung von Toluol mit SbCl₃ bekannt. Das mit diesem Katalysator beobachtete Verhältnis von o-Chlortoluol zu p-Chlortoluol beträgt 1,6:1.

Es besteht also noch immer Bedarf an einem technisch anwendbaren, katalytischen System, mit dem sich gleichzeitig eine gute Stufenselektivität und ein hoher Anteil an o-Chlorverbindungen im Reaktionsgemisch bei geringen Katalysatormengen realisieren läßt.

Es wurde nun ein Verfahren zur Kernchlorierung von aromatischen Kohlenwasserstoffen der Formel (I) 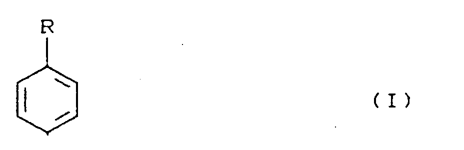
in der
- R: einen C₁-C₁₂-Alkylrest oder C₃-C₈-Cycloalkylrest bedeutet,
in Gegenwart einer Mischung von Friedel-Crafts-Katalysatoren und in Gegenwart von Co-Katalysatoren in flüssiger Phase gefunden, das dadurch gekennzeichnet ist, daß man als Friedel-Crafts-Katalysatoren eine Mischung von mindestens einer Antimon- und mindestens einer Eisenverbindung und als Co-Katalysator mindestens eine Verbindung mit Polyetherstruktur der Formel (II) 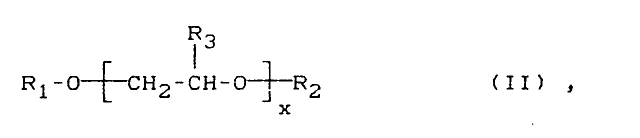
in der
- R₁ und R₂: unabhängig voneinander jeweils für Wasserstoff, einen C₁-C₁₈-Alkylrest oder C₃-C₈-Cycloalkylrest stehen oder R₁ und R₂ gemeinsam für den Rest einer ringbildenden Alkylengruppe stehen,
- R₃: Wasserstoff, Methyl oder Ethyl und
- x: eine Zahl von 1 bis 500 bedeutet,
einsetzt.

In einer bevorzugten Ausführungsform des erfindungsgemäßen Verfahrens werden als aromatische Kohlenwaserstoffe der Formel (I) Toluol, Ethylbenzol, Propylbenzol, Cumol, tert.-Butylbenzol oder Phenylcyclohexan eingesetzt.

Das erfindungsgemäße Verfahren wird in flüssiger Phase durchgeführt, d.h. der aromatische Kohlenwasserstoff der Formel (I) muß bei Reaktionstemperatur überwiegend in flüssiger Form vorliegen. Gegebenenfalls kann er zusammen mit einem inerten Lösungsmittel eingesetzt werden. Geeignete Lösungsmittel sind solche, die durch Chlor unter den Bedingungen einer Kernchlorierung nicht angegriffen werden. Beispielsweise kommen chlorierte aliphatische Kohlenwasserstoffe in Frage, wie Methylenchlorid, Chloroform und Tetrachlorkohlenstoff, sowie Essigsäure. Bevorzugt wird jedoch ohne Lösungsmittelzusatz gearbeitet.

Als Chlorierungsmittel für das erfindungsgemäße Verfahren wird vorzugsweise elementares Chlor verwendet. Dieses kann flüssig oder gasförmig in das Reaktionsgemisch eingeleitet werden. Bevorzugt wird gasförmiges Chlor eingesetzt.

Die erfindungsgemäß durchzuführende Kernchlorierung kann grundsätzlich bei beliebigen Temperaturen zwischen dem Erstarrungspunkt und dem Siedepunkt des Reaktionsgemisches durchgeführt werden. Im allgemeinen liegt die Reaktionstemperatur im Bereich zwischen 0 und 100°C, bevorzugt im Bereich zwischen 20 und 80°C, ganz besonders bevorzugt im Bereich von 40 bis 60°C. Der Druck kann während der Reaktion normal, vermindert oder erhöht sein und ist grundsätzlich unkritisch. Wegen der kostengünstigen Durchführung ist Normaldruck bevorzugt. Erhöhter Druck kann beispielsweise dann angezeigt sein, wenn in Gegenwart eines bei Normaldruck tief siedenden Lösungsmittels gearbeitet werden soll. In diesem Fall kann beispielsweise unter dem sich einstellenden Eigendruck des Reaktionsgemisches gearbeitet werden. Die Chlorierung wird bevorzugt so durchgeführt, daß der auf den zu chlorierenden aromatischen Kohlenwasserstoff bezogene Chlorierungsgrad im Reaktionsgemisch 1 nicht übersteigt. Höhere Chlorierungsgrade sind möglich, aber normalerweise nicht vorteilhaft, da sie zur Bildung meist unerwünschter mehrfach chlorierter Produkte führen.

Das Chlorierungsmittel wird daher vorzugsweise in einer Menge von 0,8 bis 1,1, bevorzugt 0,8 bis 1,0 Mol pro Mol des aromatischen Kohlenwasserstoffs eingesetzt.

Bei den Antimonverbindungen handelt es sich vorzugsweise um Antimonhalogenide, insbesondere Antimon(III)chlorid und Antimon(V)chlorid. Es kann auch elementares Antimon eingesetzt werden.

Die Menge der Antimonverbindungen als Katalysatoren kann in weiten Grenzen variiert werden. So ist bereits bei einem Zusatz von 0,0005 Gew.-% eine katalytische Wirkung erkennbar. Die Obergrenze der Katalysatormenge ist unkritisch, jedoch bieten hohe Mengen im allgemeinen hinsichtlich der Produktzusammensetzung keinen Vorteil, bringen aber häufig Schwierigkeiten bei der Aufarbeitung mit sich. Beispielsweise wird deshalb die als Katalysator eingesetzte Antimonverbindung in einer Menge von 0,001 bis 0,5 Gew.-%, bevorzugt in einer Menge von 0,01 bis 0,1 Gew.-%, eingesetzt. Alle Mengenangaben sind auf die Menge des eingesetzten aromatischen Kohlenwasserstoffs bezogen. Es kann nur eine, es können auch mehrere Antimonverbindungen, eingesetzt werden.

Bei den Eisenverbindungen handelt es sich vorzugsweise um Eisenhalogenide, insbesondere Eisen(III)chlorid. Es kann auch elementares Eisen eingesetzt werden.

Die Menge an Eisenverbindungen ist abhängig von der Menge der verwendeten Antimonverbindungen. So kann das Molverhältnis von Eisen zu Antimon in den beiden Katalysatorkomponenten z.B. in den Grenzen von Fe:Sb = 2:1 bis 1:1000 variiert werden, bevorzugt ist dieses molare Verhältnis von 1:5 bis 1:500. Es kann daher ein Eisengehalt wie er beispielsweise in der technischen Qualität des Toluols vorliegt für das erfindungsgemäße Verfahren ausreichend sein.

Erfindungsgemäß werden Co-Katalysatoren der Formel (II) eingesetzt. Bevorzugt sind Verbindungen der Formel (II), bei denen
- R₁ und R₂: jeweils unabhängig voneinander Wasserstoff, Methyl oder Ethyl bedeuten oder gemeinsam unter Ringschluß eine Ethylengruppe bilden,
- R₃: Wasserstoff oder Methyl und
- x: eine Zahl von 1 bis 10 bedeuten.

Als besondere Vertreter dieser Ausführungsform können beispielsweise 12-Krone-4-Ether, Triethylenglykoldimethylether, Triethylenglykolmonomethylether oder Glykol eingesetzt werden.

Die Menge des eingesetzten Co-Katalysators ist abhängig von der Menge des verwendeten Eisenkatalysators. So kann das molare Verhältnis von Co-Katalysator zu Eisen z.B. in den Grenzen 0,5:1 bis 5:1 variiert werden, bevorzugt ist dieses molare Verhältnis von 1:1 bis 2:1.

Die Reihenfolge der Zugabe der einzelnen Komponenten des Reaktionsgemisches kann beliebig gewählt werden. Das erfindungsgemäße Verfahren kann sowohl kontinuierlich als auch diskontinuierlich durchgeführt werden. Beispielhafte Ausführungsformen des erfindungsgemäßen Verfahrens sind folgende:
A. Ein aromatischer Kohlenwasserstoff der Formel (I), beispielsweise Toluol, wird vorgelegt und auf die gewünschte Temperatur (beispielsweise 50°C) gebracht. Dann gibt man in beliebiger Reihenfolge die gewünschten Mengen an Friedel-Crafts-Katalysator-Gemisch und Co-Katalysator zu und leitet unter weitgehender Konstanthaltung der Temperatur gasförmiges Chlor bis zum gewünschten Chlorierungsgrad ein. Anschließend wird das Gemisch durch Destillation aufgearbeitet.
B. Man stellt eine Mischung aus einem aromatischen Kohlenwasserstoff der Formel (I) mit einer Mischung der Friedel-Crafts-Katalysatoren und dem Co-Katalysator her und bringt diese auf die gewünschte Reaktionstemperatur. Dann wird Chlorierungsmittel bis zum gewünschten Chlorierungsgrad zugefügt. Die Aufarbeitung kann auch hier durch Destillation erfolgen.
C. Man stellt eine Lösung von Friedel-Crafts-Katalysator-Gemisch und Co-Katalysator in einem aromatischen Kohlenwasserstoff der Formel (I) her und führt diese Lösung einer kontinuierlich arbeitenden Chlorierapparatur zu. Man leitet ebenfalls kontinuierlich das Chlorierungsmittel so schnell ein, daß der gewünschte Chlorierungsgrad erreicht wird. Auch hier kann die kontinuierlich anfallende Reaktionsmischung durch Destillation aufgearbeitet werden.

Das erfindungsgemäße Verfahren gestattet die Herstellung von kernchlorierten aromatischen Kohlenwasserstoffen mit einer guten Stufenselektivität, einem hohen Anteil von o-Chlorverbindungen im Reaktionsgemisch und einem Einsatz von geringen Katalysatormengen. Bei der alleinigen Verwendung von Antimonkatalysatoren wird nach US 3 226 447 beispielsweise bei der Chlorierung von Toluol lediglich ein Verhältnis von o-Chlortoluol zu p-Chlortoluol von 1,6:1 erhalten. Bei zusätzlicher Zugabe von Eisen wird das o/p-Verhältnis geringfügig erhöht, gleichzeitig wird jedoch ein starker Abfall der Stufenselektivität beobachtet (siehe Beispiel 5).

### Beispiele

Prozentangaben sind, soweit nicht anders gesagt wird, Gewichtsprozente.

### Beispiel 1

In einem geschwärzten Chlorierbecher von 16 cm Höhe und 6 cm Durchmesser mit 4 Wellenbrechern wurden 200 g Toluol bei Raumtemperatur mit 65 mg SbCl₅, 24 mg FeCl₃ und 26 mg 12-Krone-4 als Co-Katalysator (entspricht einem molaren Verhältnis von Sb:Fe = 1,5:1 und Fe:Co-Katalysator = 1:1) eingewogen. Danach wurde unter leichtem N₂-Strom auf 50°C erhitzt und mit dem Einleiten von Cl₂ direkt unter dem Rührer (500 U/min) begonnen.

Die Chloriergeschwindigkeit betrug ca. 20 Mol-% pro Stunde. Nach 5 Stunden, d.h. nach dem Einleiten von 95 Mol-% Chlor, wurde dem Reaktionsgemisch eine Probe entnommen und gaschromatogaphisch untersucht. Die Analyse ergab folgende Zusammensetzung:
Toluol 7,4 %
o-Chlortoluol 66,5 %
m-Chlortoluol 2,2 %
p-Chlortoluol 21,9 %
Dichlortoluole 2,0 %
o/p-Verhältnis = 3,04

### Beispiel 2

Es wurde wie im Beispiel 1 verfahren, jedoch wurden 65 mg SbCl₅, 10 mg FeCl₃ und 11 mg Triethylenglykoldimethylether als Co-Katalysator (molares Verhältnis Sb:Fe = 7,1:1; molares Verhältnis Fe:Co-Katalysator = 1:1) eingesetzt. Die Produktzusammensetzung nach 5 Stunden war:
Toluol 5,5 %
o-Chlortoluol 67,5 %
m-Chlortoluol 2,3 %
p-Chlortoluol 21,9 %
Dichlortoluole 2,8 %
o/p-Verhältnis = 3,08

### Beispiel 3

Es wurde wie im Beispiel 1 verfahren, jedoch wurden 130 mg SbCl₅, 20 mg FeCl₃ und 21 mg Triethylenglykolmonomethylether als Co-Katalysator (molares Verhältnis Sb:Fe = 3,5:1; molares Verhältnis Fe:Co-Katalysator = 1:1) eingesetzt. Die Produktzusammensetzung nach 5 Stunden war:
Toluol 5,8 %
o-Chlortoluol 67,8 %
m-Chlortoluol 2,3 %
p-Chlortoluol 21,8 %
Dichlortoluole 2,3 %
o/p-Verhältnis = 3,11

### Beispiel 4

Es wurde wie im Beispiel 1 verfahren, jedoch wurden 130 mg SbCl₅, 0,5 mg FeCl₃ und 1,0 mg Glykol als Co-Katalysator (molares Verhältnis Sb:Fe = 141:1; molares Verhältnis Fe:Co-Katalysator = 1:5) eingesetzt. Die Produktzusammensetzung nach 5 Stunden war:
Toluol 5,7 %
o-Chlortoluol 68,5 %
m-Chlortoluol 2,2 %
p-Chlortoluol 20,9 %
Dichlortoluole 2,7 %
o/p-Verhältnis = 3,28

### Beispiel 5 (zum Vergleich)

Der in Beispiel 2 beschriebene Versuch wurde ohne Zusatz von Triethylenglykoldimethylether durchgeführt. Die Produktzusammensetzung nach 5 Stunden war:
Toluol 8,5 %
o-Chlortoluol 56,6 %
m-Chlortoluol 2,3 %
p-Chlortoluol 25,8 %
Dichlortoluole 6,8 %
o/p-Verhältnis = 2,19

## Patentansprüche

1. Verfahren zur Kernchlorierung von aromatischen Kohlenwasserstoffen der Formel (I) 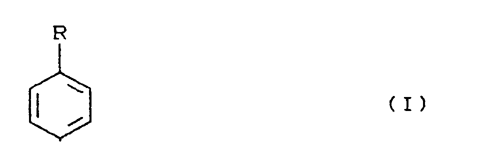 in der
R einen C₁-C₁₂-Alkylrest oder C₃-C₈-Cycloalkylrest bedeutet,
in Gegenwart einer Mischung von Friedel-Crafts-Katalysatoren und in Gegenwart von Co-Katalysatoren in flüssiger Phase, dadurch gekennzeichnet, daß man als Friedel-Crafts-Katalysatoren eine Mischung von mindestens einer Antimon- und mindestens einer Eisenverbindung und als Co-Katalysator mindestens eine Verbindung mit Polyetherstruktur der Formel (II) 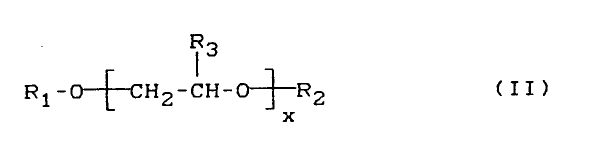 in der
R₁ und R₂ unabhängig voneinander jeweils für Wasserstoff, einen C₁-C₁₈-Alkylrest oder C₃-C₈-Cycloalkylrest stehen oder R₁ und R₂ gemeinsam für den Rest einer ringbildenden Alkylengruppe stehen,
R₃ Wasserstoff, Methyl oder Ethyl und
x eine Zahl von 1 bis 500 bedeutet,
einsetzt.

2. Verfahren nach Anspruch 1, dadurch gekennzeichnet, daß man als aromatische Kohlenwasserstoffe der Formel (I) Toluol, Ethylbenzol, Propylbenzol, Cumol, tert.-Butylbenzol oder Phenylcyclohexan einsetzt.

3. Verfahren nach Ansprüchen 1 bis 2, dadurch gekennzeichnet, daß Co-Katalysatoren der Formel (II) eingesetzt werden, worin
R₁ und R₂ jeweils unabhängig voneinander Wasserstoff, Methyl oder Ethyl bedeuten oder gemeinsam unter Ringschluß eine Ethylengruppe bilden,
R₃ Wasserstoff oder Methyl und
x eine Zahl von 1 bis 10 bedeuten.

4. Verfahren nach Ansprüchen 1 bis 3, dadurch gekennzeichnet, daß man die Chlorierung mit elementarem Chlor durchführt.

5. Verfahren nach Ansprüchen 1 bis 4, dadurch gekennzeichnet, daß man eine Reaktionstemperatur im Bereich 0 bis 100°C anwendet.

6. Verfahren nach einem der Ansprüche 1 bis 5, dadurch gekennzeichnet, daß man als Eisenverbindungen Eisen(III)chlorid oder elementares Eisen und als Antimonverbindungen SbCl₃, SbCl₅ oder elementares Antimon einsetzt.

7. Verfahren nach Ansprüchen 1 bis 6, dadurch gekennzeichnet, daß man die Antimonverbindung in einer Menge von 0,001 bis 0,5 Gew.-% bezogen auf den eingesetzten aromatischen Kohlenwasserstoff der Formel (I) einsetzt.

8. Verfahren nach Ansprüchen 1 bis 7, dadurch gekennzeichnet, daß das Molverhältnis von Eisen zu Antimon 2:1 bis 1:1000 bezogen auf eingesetztes Antimon der verwendeten Antimonverbindung beträgt.

9. Verfahren nach einem der Ansprüche 1 bis 8, dadurch gekennzeichnet, daß man den Co-Katalysator in einem molaren Verhältnis von 0,5:1 bis 5:1 bezogen auf eingesetztes Eisen einsetzt.

## Claims

1. Process for the nuclear chlorination of aromatic hydrocarbons of the formula (I) 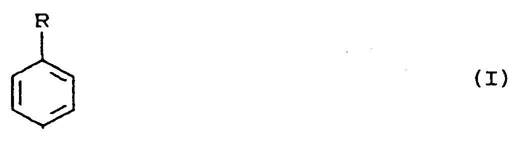 in which
R denotes a C₁-C₁₂-alkyl radical or C₃-C₈-cycloalkyl radical,
in the presence of a mixture of Friedel-Crafts catalysts and in the presence of cocatalysts in the liquid phase, characterised in that the Friedel-Crafts catalysts employed are a mixture of at least one antimony and at least one iron compound and the cocatalyst employed is at least one compound having a polyether structure of the formula (II) 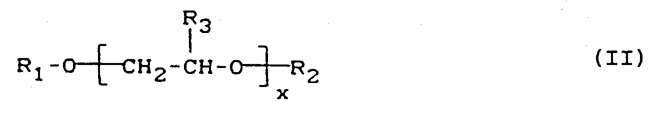 in which
R₁ and R₂ independently of one another in each case represent hydrogen, a C₁-C₁₈-alkyl radical or C₃-C₈-cycloalkyl radical, or R₁ and R₂ together represent the radical of a cyclising alkylene group,
R₃ denotes hydrogen, methyl or ethyl and
x denotes a number from 1 to 500.

2. Process according to Claim 1, characterised in that toluene, ethylbenzene, propylbenzene, cumene, tert-butylbenzene or phenylcyclohexane are employed as the aromatic hydrocarbons of the formula (I).

3. Process according to Claims 1 to 2, characterised in that cocatalysts of the formula (II) are employed, wherein
R₁ and R₂ in each case independently of one another denote hydrogen, methyl or ethyl or together, with cyclisation, form an ethylene group,
R₃ denotes hydrogen or methyl and
x denotes a number from 1 to 10.

4. Process according to Claims 1 to 3, characterised in that the chlorination is carried out using elemental chlorine.

5. Process according to Claims 1 to 4, characterised in that a reaction temperature in the range from 0 to 100°C is used.

6. Process according to one of Claims 1 to 5, characterised in that iron (III) chloride or elemental iron is employed as the iron compound and SbCl₃, SbCl₅ or elemental antimony is employed as the antimony compound.

7. Process according to Claims 1 to 6, characterised in that the antimony compound is employed in an amount of 0.001 to 0.5% by weight, based on the aromatic hydrocarbon of the formula (I) employed.

8. Process according to Claims 1 to 7, characterised in that the molar ratio of iron to antimony is 2:1 to 1:1000, based on the antimony employed in the antimony compound used.

9. Process according to one of Claims 1 to 8, characterised in that the cocatalyst is employed in a molar ratio of 0.5:1 to 5:1, based on the iron employed.

## Revendications

1. Procédé pour chlorer dans le noyau les hydrocarbures aromatiques de formule (I) 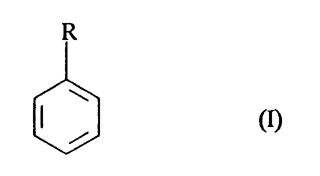 dans laquelle
R représente un groupe alkyle en C₁-C₁₂ ou cycloalkyle en C₃-C₈,
en présence d'un mélange de catalyseurs de Friedel-Crafts et en présence de catalyseurs auxiliaires, en phase liquide, caractérisé en ce que l'on utilise en tant que catalyseurs de Friedel-Crafts, en mélange entre eux, au moins un dérivé de l'antimoine et au moins un dérivé du fer, et en tant que catalyseur auxiliaire au moins un composé à structure de polyéther répondant à la formule (II) 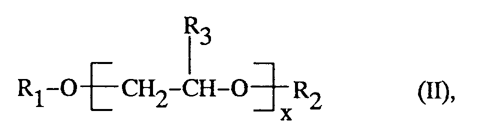 dans laquelle
R₁ et R₂ représentent chacun, indépendamment l'un de l'autre, l'hydrogène un groupe alkyle en C₁-C₁₈ ou cycloalkyle en C₃-C₈ ou bien R₁ et R₂ représentent ensemble le reste d'un groupe alkylène formant un cycle,
R₃ représente l'hydrogène, un groupe méthyle ou éthyle et
x est un nombre allant de 1 à 500.

2. Procédé selon revendication 1, caractérisé en ce que l'on met en oeuvre en tant qu'hydrocarbures aromatiques de formule (I) le toluène, l'éthylbezizène, le propylbenzène, le cumène, le tert-butylbenzène ou le phénylcyclohexane.

3. Procédé selon les revendications 1 à 2, caractérisé en ce que l'on utilise des catalyseurs auxiliaires répondant à la formule (II) dans laquelle
R₁ et R₂ représentent chacun, indépendamment l'un de l'autre, l'hydrogène, un groupe méthyle ou éthyle ou bien forment ensemble, par cyclisation, un groupe éthylène,
R₃ représente l'hydrogène ou un groupe méthyle et
x est un nombre allant de 1 à 10.

4. Procédé selon les revendications 1 à 3, caractérisé en ce que l'on exécute la chloruration avec du chlore élémentaire.

5. Procédé selon les revendications 1 à 4, caractérisé en ce que l'on observe une température de réaction dans l'intervalle de 0 à 100°C.

6. Procédé selon une des revendications 1 à 5, caractérisé en ce que l'on utilise en tant que dérivés du fer le chlorure de fer-III ou le fer élémentaire et en tant que dérivés de l'antimoine SbCl₃, SbCl₅ ou l'antimoine élémentaire.

7. Procédé selon les revendications 1 à 6, caractérisé en ce que l'on met en oeuvre le dérivé de l'antimoine en quantité de 0,001 à 0,5 % du poids de l'hydrocarbure aromatique de formule (I) soumis à la chloruration.

8. Procédé selon les revendications 1 à 7, caractérisé en ce que le rapport molaire fer/antimoine va de 2 : 1 à 1 : 1 000 pour ce qui concerne l'antimoine du dérivé d'antimoine mis en oeuvre.

9. Procédé selon l'une des revendications 1 à 8, caractérisé en ce que l'on met en oeuvre le catalyseur auxiliaire en quantité correspondant à un rapport molaire de 0,5 : 1 à 5 : 1 avec le fer mis en oeuvre.
